# EUROPÄISCHE PATENTSCHRIFT

(11) **EP 3 223 699 B1**
(45) Veröffentlichungstag und Bekanntmachung des Hinweises auf die Patenterteilung: **12.09.2018**
(21) Anmeldenummer: 15813255.5
(22) Anmeldetag: 24.11.2015
(51) Int. Cl.: A61B 5/055, G01R 33/34

(54) **PATIENTENLIEGE FÜR EINE KERNRESONANZTOMOGRAPHIE-UNTERSUCHUNG**
PATIENT EXAMINATION TABLE FOR A NUCLEAR RESONANCE TOMOGRAPHY EXAMINATION
TABLE MÉDICALE POUR UN EXAMEN PAR TOMOGRAPHIE PAR RÉSONANCE NUCLÉAIRE

(30) Priorität: 25.11.2014 DE 102014117274
(43) Veröffentlichungstag der Anmeldung: 04.10.2017
(73) Patentinhaber: Noras, Hubert, 97082 Würzburg (DE)
(72) Erfinder: Noras, Hubert, 97082 Würzburg (DE)
(74) Vertreter: Pöhner, Wilfried Anton
(86) Internationale Anmeldenummer: PCT/DE2015/100500
(87) Internationale Veröffentlichungsnummer: WO 2016/082824

(56) Entgegenhaltungen:
- US-A1- 2010 041 979
- US-A1- 2010 099 978
- US-A1- 2014 128 723
- US-A1- 2014 213 886
- US-A1- 2014 296 701

## Beschreibung

Die Erfindung betrifft eine Patientenliege für eine Kernresonanztomographie-Untersuchung eines Körperteils eines Patienten, insbesondere der Mamma, umfassend zwei Aussparungen, welche jeweils für die Aufnahme einer Mamma bestimmt sind, und in deren unmittelbarer Nähe eine Untersuchungseinrichtung angeordnet ist.

Ein Mammakarzinom, also ein Brustkrebs, ist der häufigste bösartige Tumor der Mamma des Menschen, welcher hauptsächlich bei Frauen, aber selten auch bei Männern vorkommt. Es ist daher nicht verwunderlich, dass ein Mammakarzinom in den westlichen Staaten die häufigste Krebsart bei Frauen ist, an welcher mehr Frauen als an irgendeiner anderen Krebserkrankung sterben. In den meisten Fällen tritt die Erkrankung dabei sporadisch auf, wobei aber sowohl erbliche als auch erworbene Risikofaktoren existieren.

Der Vorsorgeuntersuchung eines Mammakarzinoms kommt daher fundamentale Bedeutung zu, um die Mortalität aufgrund einer Früherkennung und strukturierten Behandlung zukünftig zu senken. Als bildgebende Untersuchungsverfahren werden in der Röntgenmammographie Röntgenstrahlen, in anderen Untersuchungsverfahren Ultraschall (Sonographie) und in letzter Zeit immer häufiger Kernspintomographieverfahren eingesetzt. Bei der Kernspinresonanztomographie oder auch Magnetresonanztomographie (MRT) hat sich herausgestellt, dass die Sensitivität des Nachweises krebsartiger Gewebe höher ist als bei der Untersuchung mit Röntgenstrahlen oder Ultraschall.

Ein MRT ist ein nicht-invasives, bildgebendes Verfahren, das vor allem in der medizinischen Diagnostik zur Darstellung von Struktur und Funktion der Gewebe und Organe im Körper eingesetzt wird. Somit werden mittels eines MRTs Schnittbilder eines Körpers erzeugt, die eine Beurteilung der Organe und vieler krankhafter Organveränderungen erlauben. Das MRT-Untersuchungsprinzip basiert im Wesentlichen auf in einem MRT-Gerät erzeugten, sehr starken Magnetfeldern sowie magnetischen Wechselfeldern im Radiofrequenzbereich, mit welchen meist die Wasserstoffkerne/Protonen im Körper resonant angeregt werden, die zu einer Anregung der Spinenergie führen. Mit Relaxation, d.h. nach Rückkehr in seinen Ausgangszustand, wird von dem Spin eine Radiowelle emittiert, die durch geeignete Detektoren erfasst wird. Je größer die Anzahl der Protonen ist, wie sie insbesondere bei wasserhaltigem Gewebe in erhöhtem Maße vorkommen, umso höher ist die emittierte und gemessene Signaldichte. Folglich lassen sich mittels eines MRTs anatomische Anomalien erkennen und beurteilen.

Typischerweise werden bei Untersuchungen der Mamma, d.h. der weiblichen oder männlichen Brustdrüse eines Patienten, zusätzlich Kontrastmittel appliziert, die sich in besonderer Weise dort anreichern, wo ein erhöhter Blutfluss herrscht und die zu einer wesentlichen Verbesserung der Bildaufnahmequalität und deren Aussagekraft führen.

Bei der Mammographie wird in der Regel jede Mamma aus zwei, gegebenenfalls auch aus mehreren Richtungen aufgenommen, wobei die cranio-caudale Abbildung (Röntgenbild der Mamma von oben) und die mediolateral-oblique Aufnahme (Röntgenbild der Mamma von der Mitte nach außen) die am häufigsten verwendeten Abbildungen sind. Problematisch ist jedoch, dass die Mamma während der Aufnahme zwischen dem Objekttisch und einer Plexiglasplatte moderat komprimiert wird, um die Strahlendosis gering zu halten und das zu untersuchende Mammagewebe bestmöglich abzubilden. Diese Komprimierung wird von den meisten Patientinnen als unangenehm und/oder sogar schmerzhaft empfunden.

Auf aktuellem Stand der Technik sind daher verschiedene Möglichkeiten zur Weiterentwicklung und/oder Verbesserung derartiger MRT-Geräte zur Mammographie bekannt, um vor allem die Bildqualität der Aufnahme zu erhöhen. Zudem ist die Anordnung der Spulen bezüglich des Zentrums des Untersuchungsvolumens wichtig, wie der Patentschrift DE 44 19 454 B4 zu entnehmen ist. Die Patentschrift schlägt dabei vor, dass koaxial zu jeder Magnetspule jeweils zwei ferromagnetische Ringe vorgesehen sind, welche derart positioniert und dimensioniert sind, dass das Magnetfeld im Zentrum des Untersuchungsvolumens mindestens bis zur 10. Ordnung homogenisiert wird.

Die Offenlegungsschrift EP 0 583 824 A2 betrifft eine Spulenanordnung für MR-Untersuchungen der Mamma. Die erste Spulenanordnung umfasst auf der Mantelfläche eines Zylinders in zueinander parallelen Ebenen mehrere lediglich induktiv miteinander gekoppelte Leiterschleifen, von denen eine an einen Eingang einer Empfängerspule anschließbar ist und mit einer als Oberflächenspule vom Butterflytyp ausgebildeten zweiten Spulenanordnung, die sich an einer der Stirnflächen der ersten Spulenordnung befindet, an einen weiteren Eingang der Empfängeranordnung anschließbar ist. Auf diese Weise wird das Signal-Rausch-Verhältnis verbessert.

Die Offenlegungsschrift EP 0 486 086 A1 schlägt eine Quadraturspulenanordnung für Kernspinuntersuchungsgeräte vor, deren homogenes stationäres Magnetfeld senkrecht zur Längsachse eines darin untersuchten Patienten verläuft und nicht nur bei Kopf- oder Extremitätenuntersuchungen einsetzbar ist. Dabei enthält die erste Hochfrequenzspulenanordnung mehrere lediglich induktiv miteinander gekoppelte, in Bezug auf die Symmetrieachse gegeneinander versetzte ringförmige Leiterschleifen. Die zweite Spulenanordnung enthält bogenförmig umschließende Leiter, die über zur Symmetrieachse parallele Leiter miteinander verbunden sind.

Nachteilig bei den im Stand der Technik bekannten Spulenanordnungen ist jedoch, dass die Mammographie aufgrund der Vielzahl der abzubildenden Ebenen relativ lange dauert. Ein weiterer Nachteil der bekannten Systeme besteht darin, dass die Spulen und der für die Entnahme einer Probe erforderliche Biopsierahmen baulich voneinander getrennt sind, so dass die Probenentnahme mit schlechterer Qualität als die Befundung durchgeführt wird.

Um diesem Problem zu entgehen, schlägt die Offenlegungsschrift DE 195 28 260 A1 eine Mammographie-Antenne für Magnetresonanzuntersuchungen mit einem Untersuchungsraum vor, welche in einem Halteteil zwei parallel und gegeneinander bewegliche, ebene Kompressionsplatten aufweist. Die Kompressionsplatten können wiederum mit Bohrungen für die Führung einer Biopsienadel versehen sein.

Nachteilig bei der auf aktuellem Stand der Technik bekannten Mammographie-Antenne ist jedoch, dass die Kompressionsplatten eben ausgestaltet sind und sich daher nicht optimal an die Mamma anpassen, so dass die Mammographie aufgrund der erforderlichen Kompression der Mamma für den Patienten nicht nur unangenehm bis teilweise schmerzhaft ist und zu dem aufgrund der Vielzahl der abzubildenden Ebenen auch lange dauert.
In der Offenlegungsschrift US 2014/0213886 A1 wird versucht ein gewisses Maß an anatomischer Konformität dadurch herzustellen, dass die die Mammae umfassenden Spulenvorrichtungen eine schwach konkave Krümmung aufweisen.
Es besteht daher ein großer Bedarf an einer Patientenliege für eine Kernresonanztomographie-Untersuchung, insbesondere der Mamma, mittels welcher das Untersuchungsvolumen auf kleinstem Querschnitt reduziert ist und mittels welcher die Probenentnahme mit der gleichen Qualität wie die Befundung durchführbar ist.
Die Erfindung hat sich somit die Aufgabe gestellt, eine Patientenliege für eine Kernresonanztomographie-Untersuchung, insbesondere der Mamma, bereitzustellen, um die oben genannten Schwierigkeiten zu überwinden und um vor allem die Untersuchung, und gegebenenfalls eine Probenentnahme, in möglichst kurzer Zeit bei geringer Strahlenbelastung und bestmöglicher Bildqualität durchführen zu können. Diese Aufgabe wird auf überraschend einfache, aber wirkungsvolle Weise durch eine Patientenliege gemäß Anspruch 1 gelöst.

Es wird eine Patientenliege für eine Kernresonanztomographie-Untersuchung eines Körperteils eines Patienten, insbesondere der Mamma, vorgeschlagen, welche zwei Aussparungen für die Aufnahme des Körperteils umfasst, in deren unmittelbarer Nähe eine Untersuchungseinrichtung angeordnet ist.
Unter dem Begriff "Patientenliege" versteht sich eine Liege für einen Patienten, auf welcher der Patient liegt. Dabei wird ein Körperteil des Patienten, insbesondere die Mamma, untersucht. Vom Grundsatz her ist die Patientenliege dabei nicht auf die Untersuchung der Mamma bzw. der Mamma beschränkt, sondern kann auch für Untersuchungen anderer Körperteile verwendet werden. Bei der Untersuchung einer und/oder beider Mammae liegt der Patient bäuchlings auf der Patientenliege und die Mammae werden jeweils in eine Aussparung eingefädelt, welche jeweils für die Aufnahme einer Mamma bestimmt sind. Dabei wird die Mamma unterhalb der Liege untersucht. Zu diesem Zweck ist in unmittelbarer Nähe des zu untersuchenden Gewebes eine Untersuchungseinrichtung angeordnet, welche Spulen, insbesondere Empfangsspulen, für die emittierten Radiowellen und Biopsieeinheiten für eine optionale Probenentnahme umfasst.

Unter einer "Untersuchungseinrichtung" versteht sich eine Einrichtung zur Untersuchung eines Gewebes, beispielsweise der Mamma im Rahmen einer Mammographie, welche unterhalb der Patientenliege in unmittelbarer Nähe des zu untersuchenden Gewebes, insbesondere der Mamma, angeordnet ist. D.h. dass die Untersuchungseinheit so nah zu dem untersuchenden Gewebe angeordnet ist, dass diese das Gewebe berührt. Eine derartige Nähe zu dem zu untersuchenden Gewebe ist wichtig, da nur bei unmittelbarer Nähe der von den Spulen emittierten oder detektierten Radiowellen, welche von der Untersuchungseinrichtung umfasst sind, eine hohe Bildqualität sichergestellt werden kann.

Im Rahmen der Erfindung umfasst die Untersuchungseinrichtung eine mediale Spule, eine laterale Spule, eine mediale Biopsieeinheit und eine laterale Biopsieeinheit. Es versteht sich dabei, dass die Untersuchungseinrichtung nicht nur für eine Untersuchung eines Gewebes geeignet ist, sondern auch für therapeutische Zwecke, wie beispielsweise eine Magnetfeldtherapie. Dabei wird nicht der Anspruch gestellt, dass eine Therapie zu 100% erfolgreich sein muss.

Der Begriff "Spule" ist dem Fachmann bekannt und betrifft in der Elektrotechnik Wicklungen und Wickelgüter, die geeignet sind, ein Magnetfeld zu erzeugen und/oder zu detektieren. In der Regel besteht eine Spule aus mindestens einer Wicklung eines Stromleiters, wie beispielsweise Draht, Kupferlackdraht, versilbertem Kupferdraht oder Hochfrequenzlitze, welcher meist auf einem Spulenkörper bzw. Spulenträger gewickelt ist und überwiegend mit einem magnetischen Kern versehen ist. Im Rahmen der Erfindung kann eine Spule daher eine Sendespule zum Aussenden von Radiowellen und/oder eine Empfangsspule zur Detektion emittierter Radiowellen sein. Die Spule kann wenigstens ein Spulenelement, bevorzugt zwei, drei, vier oder mehr Spulenelemente aufweisen.
Unter einer "Biopsieeinheit" versteht sich eine Einheit, welche für verschiedene Formen der Gewebe- und/oder Probenentnahme des untersuchten Gewebes geeignet ist. Die Einheit umfasst zumindest eine Spezialkanüle, bspw. eine Biopsienadel, und weist wenigstens eine Aussparung für die Einführung derselben auf. Dabei ist die Einheit derart ausgestaltet, dass eine Entnahme und/oder Untersuchung von Gewebe ohne Sichtkontrolle durch Eingabe der mittels Kernresonanztomographie ermittelten Koordinaten und/oder in Kombination mit bildgebenden Verfahren durchgeführt wird. Die Biopsie ist dabei manuell und/oder maschinell betrieben durchführbar.
Es ist dabei erfindungswesentlich, dass die Spulen und die Biopsieeinheiten senkrecht zur Körperoberfläche des Patienten ausgerichtet sind. Die senkrechte Ausrichtung ist wichtig, damit die Spulen und die Biopsieeinheit das zu untersuchende Gewebe vollständig und allseitig umschließen, d.h. von allen Seiten umrahmen, und somit auch fixieren. Es kann jedoch bei besonders großen Mammae vorkommen, dass diese aufgrund ihres eigenen Platzbedarfs nicht komplett umschlossen werden können. In einem solchen Fall ist es auch ausreichend, wenn die Mamma nahezu vollständig umschlossen ist, da aufgrund der Größe der Mamma ausreichend Gewebe für eine Befundung und/oder Biopsie vorhanden ist. Die Grundlagen der erfindungsgemäßen Vorschläge bestehen dabei in der Erkenntnis, dass eine allseitige Fixierung der Mamma sichergestellt ist, so dass es zu keinen Relativbewegungen des Messobjektes während der Dauer der Aufnahme und demzufolge zu Unschärfen in der Bildqualität kommt.

Zudem kann das zu untersuchende Gewebe aufgrund der allseitig umschließenden und fixierenden Untersuchungseinrichtung zugleich komprimiert werden, d. h., dass das zu untersuchende Gewebe entsprechend eingeengt wird. Dadurch kommt es zu einer Erhöhung der Bildqualität für die Befundung. Zudem verringert sich die Anzahl der aufzunehmenden Schichten, unabhängig davon, ob sie in transversaler oder sagittaler Richtung verlaufen, aufgrund der Kompressionen der Mamma auf ein Minimum. Je geringer die Anzahl der aufzunehmenden Schichten zur Abbildung des gesamten Objektes ist, umso schneller lässt sich die Aufnahme durchführen. Wird der aufzunehmende Gewebebereich in seinen Abmessungen stark verkleinert, kann die Aufnahme auch schneller durchgeführt werden als es bei der Aufnahme von Mammae größerer Abmessungen der Fall wäre.

Es ist des Weiteren erfindungswesentlich, dass die mediale Spule und die mediale Biopsieeinheit verbunden sind, miteinander in Wirkverbindung stehen und eine mediale Untersuchungseinheit ausbilden, wobei diese mittig in der Untersuchungseinrichtung und zwischen den Mammae angeordnet ist. Es ist zudem wichtig, dass die laterale Spule und die laterale Biopsieeinheit verbunden sind, miteinander in Wirkverbindung stehen und eine laterale Untersuchungseinheit ausbilden, wobei diese seitlich in der Untersuchungseinheit und der Mammae angeordnet ist.

Im Regelfall sind die mediale und die laterale Untersuchungseinheit gleich ausgelegt und/oder ausgebildet, wobei ein geringfügiger Unterschied aufgrund der Anpassung an die Größe des Zugangs der Patientenauflage hinsichtlich der lateralen Untersuchungseinheit bestehen kann.

Dabei haben die Spulen innenliegend Kontakt mit der Mamma, wobei die Biopsieeinheiten lediglich über die Spulen in Kontakt mit der Mamma stehen. Das bedeutet, dass die Spule und die Biopsieeinheit eine bauliche Einheit bilden, d.h. sie sind auf irgendeine Weise miteinander verbunden, so dass eine gegebenenfalls notwendige Gewebe- und/oder Probenentnahme aus der Mamma mit der gleichen hohen Qualität wie die Befundung durchgeführt wird. Für eine etwaige Gewebeentnahme ist es allerdings wichtig, dass ausreichend zu biopsierendes Gewebe vorhanden ist, so dass sich auch hier die enorme Wichtigkeit der Komprimierung des zu untersuchenden Gewebes zeigt. Aufgrund der Komprimierung kommt es nicht nur zu einer weiteren Reduktion der Mammographie-Dauer, sondern auch zu einer Verbesserung der Bildqualität für die Befundung. Die bei der Befundung mittels Kernresonanztomographie ermittelten Koordinaten einer zu entnehmenden Probe können dann im nächsten Schritt direkt und unmittelbar, d.h. ohne erneute Einstellung der Koordinaten und ohne Wiederholung der Untersuchung, sowie bei gleichhoher Qualität wie die Befundung entnommen werden können. Damit erhöht sich auch die Qualität der biopsierten Gewebeprobe.

Hierbei ist ein weiterer wesentlicher Vorteil auch darin zu sehen, dass es für eine präzise und zielgerichtete Entnahme einer Gewebeprobe mittels einer Spezialkanüle wichtig ist, dass das zu entnehmende Gewebe fixiert ist. Erst der fixierte Zustand erlaubt somit eine besonders präzise und zielgerichtete Entnahme des jeweiligen Gewebes.

Es ist des Weiteren erfindungswesentlich, dass die mediale und die laterale Untersuchungseinheit an die zu untersuchende Mamma angepasst sind, wobei wenigstens die sich gegenüberliegenden Enden der Spulen in Richtung der Mamma abgewinkelt und/oder gebogen sind. Verständlicherweise ist es auch möglich, dass die komplette Untersuchungseinheit, d.h. die Spule und die Biopsieeinheit, abgewinkelt und/oder gebogen ist.

Aufgrund der Winkelung und/oder Biegung passen sich die Untersuchungseinheiten an die natürliche Form der Mamma optimal an, was nochmal zu einer Reduzierung des Untersuchungsvolumens der Mamma führt. Im Rahmen der Erfindung ist erkannt worden, dass die zusätzliche Reduzierung des Untersuchungsvolumens auf kleinsten Querschnitt zahlreiche Vorteile mit sich bringt. Zum einen verringert sich die Anzahl der notwendigen Aufnahmen des Mammagewebes deutlich, was nicht nur mit einer Verringerung der Strahlenbelastung des untersuchten Gewebes, sondern auch mit einer starken Verkürzung der Dauer der Mammographie einhergeht. Des Weiteren ist eine verringerte Dauer besonders erstrebenswert, da die für die bestmögliche Abbildung der Mamma notwendige Gewebekompression von den meisten Patientinnen als unangenehm und/oder sogar schmerzhaft empfunden wird. Gemäß der Erfindung ist die Winkelung und/oder Biegung der medialen und/oder lateralen Untersuchungseinheit beweglich und/oder feststellbar Indem die die Winkelung und/oder Biegung beweglich ist, wird eine noch bessere Anpassung an die natürliche Form der Mamma, insbesondere an unterschiedlich große Mamma, erreicht. Aufgrund der Feststellung der Winkelung und/oder Biegung wird dabei sichergestellt, dass sich die Fixierung und/oder Komprimierung der Mamma nicht wieder löst, um somit optimale Voraussetzungen für eine Kernresonanztomographie-Untersuchung und/oder gegebenenfalls eine Biopsie zu schaffen.
Verständlicherweise ist es möglich, die Winkelung und/oder Biegung der medialen und der lateralen Untersuchungseinheit unabhängig voneinander und/oder unterschiedlich zu bewegen bzw. einzustellen. Dies ist insbesondere wichtig, um die Untersuchungseinrichtung an unterschiedlich große Mammae anzupassen.
Es hat sich dabei als erfindungswesentlich herausgestellt, dass die Winkelung und/oder Biegung der Untersuchungseinheiten 1° bis 50° beträgt, um die Mamma optimal innerhalb der Untersuchungseinrichtung zu fixieren und/oder zu komprimieren. Bevorzugt beträgt die Winkelung und/oder Biegung dabei 10° bis 45°, noch mehr bevorzugt 20° bis 35° und am meisten bevorzugt ungefähr 30°. Alternativ ist es auch möglich, die Winkelung und/oder Biegung stufenlos anhand der mittels Kernresonanztomographie ermittelten Messdaten einzustellen, und zwar auf einen Winkel, bei welchem ein guter Empfang sichergestellt ist. Dabei wird der Winkel und/oder die Biegung so lange verändert, bis das Signal maximal ist.

Vorteilhafte Weiterbildungen der Erfindung, welche einzeln oder in Kombination realisierbar sind, sind in den Unteransprüchen dargestellt.

In einer Weiterbildung ist die Winkelung und/oder Biegung der medialen und/oder lateralen Untersuchungseinheit manuell und/oder maschinell betrieben, insbesondere mechanisch, pneumatisch und/oder elektrisch, einstellbar. Dadurch wird die individuelle Anpassung der Untersuchungseinrichtung an die zu untersuchende Mamma erleichtert. Eine derartige Anpassung ist insbesondere für Patienten mit unterschiedlich großen Mammae wichtig.
Dem Fachmann sind die unterschiedlichen Lösungen für eine mechanische, pneumatische und/oder elektrische Winkelung und/oder Biegung bekannt.
Weiterhin bevorzugt umfasst die mediale und/oder laterale Untersuchungseinheit wenigstens einen Pointer und/oder Marker. Bevorzugt umfasst die Untersuchungseinheit dabei einen Pointer und/oder Marker, noch mehr bevorzugt zwei, drei, vier oder mehr Pointer und/oder Marker. Am meisten bevorzugt umfassten beide Untersuchungseinheiten wenigstens einen Pointer und/oder Marker, bevorzugt zwei, drei, vier oder mehr Pointer und/oder Marker.
Ein Pointer und/oder Marker ist mit einer in der Kernresonanztomographie sichtbaren Substanz und/oder Flüssigkeit, beispielsweise einem Kontrastmittel, gefüllt und dient der Markierung des untersuchten Gewebes. Der Pointer und/oder Marker ist durch die Bildgebung in drei Ebenen in einem festen Winkel sichtbar, um auf diese Weise optimal die Position einer Befundung für eine gegebenenfalls nachfolgende Biopsie zu markieren.

Der Pointer und/oder Marker ist dabei derart ausgestaltet, dass er fest oder lösbar, d.h. abnehmbar, mit der Untersuchungseinheit verbunden ist. Im Regelfall wird der Pointer und/oder Marker auf der Außenseite der Biopsieeinheit, d.h. außerhalb der Untersuchungseinheit und in Richtung der Mamma, angebracht. Die erste Alternative ist aufsteckbar und befestigbar.

In einer alternativen Ausgestaltung ist die mediale und/oder laterale Untersuchungseinheit, insbesondere deren Aufnahme und/oder Führung, in der Position drehbar und/oder einstellbar. Das bedeutet, dass jede Untersuchungseinheit unabhängig von der anderen Untersuchungseinheit in der Position gedreht und/oder eingestellt werden kann. Dabei ist es im Rahmen der Erfindung vorgesehen, dass die Untersuchungseinheit, insbesondere deren Aufnahme und/oder Führung, um bis zu 360° drehbar und/oder in alle Richtungen stufenlos einstellbar ist. Auf diese Weise wird eine optimale Anpassung an die Form der entsprechenden Mamma erreicht und somit eine bestmögliche Fixierung und/oder Komprimierung der Mamma sichergestellt.

Die Wichtigkeit der stufenlosen Einstellung in alle Richtung zeigt sich besonders bei unterschiedlich großen Mammae. So müssen die medialen und/oder lateralen Untersuchungseinheiten in der Regel bei einer kleinen Mamma sehr weit zusammengeschoben und/oder gegebenenfalls in deren Aufnahme und/oder Führung gedreht werden, um genügend zu untersuchendes Gewebe zu fixieren und/oder zu komprimieren. Im Gegensatz ist es bei besonders großen Mammae erforderlich, die medialen und/oder lateralen Untersuchungseinheiten möglichst weit voneinander zu entfernen, um genügend Platz für die Mamma zu schaffen.

Dabei sind die mediale und die laterale Untersuchungseinheit, insbesondere deren Spulen, so aufeinander abgestimmt, dass bei einem Zusammenschieben bis zu einer vorgegebenen Position und/oder der Mamma geeigneten und angepassten Position keine Artefakte in der Bildgebung entstehen können. Das ist besonders wichtig, um eine hohe Bildqualität auch bei Frauen mit kleinen Mammae sicherzustellen.

Ein weiterer Vorteil einer drehbaren und/oder einstellbaren Untersuchungseinheit liegt darin, dass durch Drehung und/oder Veränderung der Einstellung diese das zu untersuchende Gewebe in einer von allen Seiten gut zugänglichen Form bewegt werden kann. Somit kann es auch während einer Untersuchung mechanisch und/oder motorisch gelöst und in eine bessere Zugangsposition für eine Biopsie gebracht werden.

In einer Ausgestaltung der Erfindung umfasst die Untersuchungseinrichtung eine Stützspule, welche von außen und unten auf die Untersuchungseinrichtung aufsteckbar ist. Die aufsteckbare Stützspule verhindert somit zuverlässig ein Durchhängen der Mammae durch die Untersuchungseinheit und verbessert die Fixierung und/oder Komprimierung des Gewebes.

Die Stützspule der Untersuchungseinrichtung ist dabei Waagerecht in einem Winkel von 90° zu den senkrecht zu der Körperoberfläche des Patienten angeordneten Spulen der Untersuchungseinheit angeordnet. Zudem ist die Stützspule in Richtung der Mamille, d.h. der Brustwarze, ausgerichtet. Die Stützspule kann dabei wenigstens ein Spulenelement, bevorzugt zwei, drei, vier oder mehr Spulenelemente aufweisen.

In noch einer Ausgestaltung ist die Stützspule senkrecht in Richtung der Patientenliege und in Richtung auf die Mamille zu bewegbar und/oder abnehmbar.

Bevorzugt ist die Stützspule dabei so ausgebildet, dass sie in den Freiraum zwischen den beiden Spulen in Richtung des zu untersuchenden Gewebes in die gewünschte und/oder vorgegebene Position gebracht werden kann. Diese Bewegung in die Position kann manuell und/oder elektrisch, beispielsweise mechanisch, pneumatisch und/oder motorisch, erfolgen. Die Bewegung ist dabei stufenlos einstellbar und mittels geeigneter Einrichtungen durchführbar. Um eine bestmögliche Bildqualität sicherzustellen, sind die Stützspulen elektronisch entsprechend abgestimmt.

Durch die Bewegung der Stützspule in Richtung der Patientenliege und in Richtung der Mamille wird erreicht, dass die Stützspule zu einer zusätzlichen Fixierung und/oder Komprimierung der Mamma beiträgt, um vor allem die Bildqualität für die Befundung und/oder gegebenenfalls Biopsie zu verbessern. Auf diese Weise wird erreicht, dass bei einer Untersuchung der Mamma eine hochauflösende Bildgebung auch bei kleinen Mammae in Richtung der Mamille gegeben ist.

Verständlicherweise ergibt es sich, dass eine derartige Stützspule besonders bei kleinen Mammae notwendig ist. Bei besonders großen Abmessungen der Mammae, bei welchen aufgrund der medialen und/oder lateralen Fixierung und/oder Komprimierung bereits ausreichend zu untersuchendes Gewebe vorhanden ist, kann auf die zusätzliche Stützspule verzichtet werden. Daher ist es wichtig, dass diese abnehmbar ausgestaltet ist, um die erfindungsgemäße Vorrichtung einfach und schnell an die Bedürfnisse unterschiedlicher Patienten anzupassen.

In einer Alternative umfasst die Untersuchungseinrichtung zusätzlich eine Ringspule, welche von unten und außen auf die Untersuchungseinrichtung aufsteckbar ist, horizontal am Körper des Patienten anliegt und sich in Richtung dessen Kopfes erstreckt. Die Ringspule ist bevorzugt eine flach ausgestaltete Spule, um es zu ermöglichen, dass sie horizontal am Körper des Patienten anliegt. Dabei ist die Ringspule bevorzugt im oberen Bereich der Untersuchungseinheit angeordnet, so dass sie sich lediglich in Richtung des Kopfes des Patienten erstreckt. Die Ringspule kann wenigstens ein Spulenelement, bevorzugt zwei, drei, vier oder mehr Spulenelemente aufweisen.

Bevorzugt wird die Untersuchungseinheit mit der aufgesteckten Ringspule für die Untersuchung eines Patienten mit kleinen Mammae eingesetzt, um so bessere Aufnahmen des zu untersuchenden Gewebes zu erreichen.

Eine weitere Ausgestaltung schlägt vor, dass die Untersuchungseinrichtung ein erstes Zusatz-Spulenelement umfasst, welches seitlich in Richtung der Axillae auf die Untersuchungseinrichtung aufsteckbar ist. Das erste Zusatz-Spulenelement kann wiederum wenigstens ein Spulenelement, bevorzugt zwei, drei, vier oder mehr Spulenelemente aufweisen.

Mittels des ersten Spulenelementes ist es erstmals möglich, zusammen mit der Untersuchung der Mammae auch die Axilla, d.h. die Achsel, und vor allem die in der Achselregion befindlichen Achsellymphknoten zu untersuchen. Die Untersuchung der Lymphknoten der Achsel ist besonders wichtig, da diese meist der erste Ort sind, an dem sich bei einem positiv befundeten Mammakarzinom Metastasen bilden. Um diesen Befall zu erfassen, werden die Achsellymphknoten, zumindest einige von ihnen, bei der Untersuchung der Mammae gleich mit untersucht.

In der Weiterbildung der Erfindung ist es vorgesehen, dass die Ringspule und/oder das erste Zusatz-Spulenelement abnehmbar ist. Bei einer Untersuchung eines Patienten mit großen oder besonders großen Mammae ist es sinnvoll auf die obere horizontal am Körper anliegende Ringspule und/oder das erste Zusatz-Spulenelement zu verzichten, da bereits ausreichendes fixiertes und/oder komprimiertes Gewebe für eine Untersuchung vorhanden ist.

Zudem wird Platz für die Mamma in der Untersuchungseinrichtung geschaffen, insbesondere indem die medialen Untersuchungseinheiten nach dem Abnehmen der Ringspule am Sternum, d.h. am Brustbein des Patienten, miteinander verbunden und/oder gekoppelt werden. Die Verbindung und/oder Koppelung erhöht zudem auch die Stabilität der Untersuchungseinrichtung.

In noch einer Weiterführung der Erfindung umfasst die Untersuchungseinrichtung ein zweites Zusatz-Spulenelement, mittels welchem die medialen Untersuchungseinheiten am Sternum miteinander verbindbar und/oder koppelbar sind. Das zweite Zusatz-Spulenelement kann ebenfalls wenigstens ein Spulenelement, bevorzugt zwei, drei, vier oder mehr Spulenelemente aufweisen.

Die Verbindung und/oder Koppelung der medialen Untersuchungseinheiten führt zu einer Erhöhung der die Stabilität der Untersuchungseinrichtung.

Die Weiterentwicklung der Weiterführung sieht vor, dass das zweite Zusatz-Spulenelement für eine bestmögliche Anpassung an den Patienten und/oder das zu untersuchende Körperteil in Richtung des Sternums des Patienten bewegbar, winkelbar, biegbar und/oder abnehmbar ausgestaltet ist. Dadurch wird nicht nur eine optimale Anpassung der Untersuchungseinheit an die Form des zu untersuchenden Gewebes erreicht, sondern auch, dass der Patient auf dem zweiten Zusatz-Spulenelement abgestützt werden kann.

Viele Patienten empfinden es als unangenehm und/oder teilweise schmerzhaft, dass sich nahezu das gesamte Körpergewicht ausschließlich über das Sternum auf der Unterlage abstützt. Daher empfiehlt es sich, das zweite Zusatz-Spulenelement für eine bestmögliche Anpassung in einer senkrechten Ausrichtung zur Patientenliege und außerhalb des Sternums nach oben bewegbar anzubringen und den Körper auch hierdurch zu unterstützen. Die Unterstützung des Körpers des Patienten basiert auf einer Verteilung des Kraftflusses und einer Verminderung der punktuellen Druckbelastung. Derartige Unterstützungen werden von den meisten Patienten als angenehm empfunden, insbesondere bei Patienten mit besonders großen Abmessungen der Mammae.

In dem Fall von besonders großen Mammae kann es allerdings auch notwendig sein, das zweite Zusatz-Spulenelement von der Untersuchungseinrichtung abzunehmen. Dann entsteht mehr Raum für die Einstellung der beiden Untersuchungseinheiten.

In einer alternativen Ausgestaltung der Erfindung umfasst die Untersuchungseinrichtung wenigstens eine Öffnung und/oder einen Schlitz, in welche ein Stab, ein Band und/oder eine Platte einbringbar und/oder anbringbar ist.

Die Öffnung und/oder der Schlitz erlaubt die Einbringung und/oder Anbringung eines sterilen, medizinischen Gerätes, beispielsweise in Form eines Stabes, eines Bandes und/oder einer Platte. Auf diese Weise wird erreicht, dass das zu untersuchende Gewebe in einem Winkel von ca. 90° zu den Untersuchungseinheiten senkrecht bzw. bei Drehung der Untersuchungseinheiten waagerecht immer zur Öffnung und/oder dem Schlitz hin frei ist. Dadurch wird das zu untersuchende Gewebe in einer geeigneten Form zur Diagnose und/oder gegebenenfalls zu einer späteren Biopsie gebracht.

Mittels des Stabes, des Bandes und/oder der Platte ist es möglich, die Mamma entsprechend über die Winkelung und/oder Biegung zu positionieren, indem beispielsweise bei einer großen Mamma mit dem Stab, dem Band und/oder der Platte das zu untersuchende und gegebenenfalls aus der Untersuchungseinrichtung herausquellende Mammagewebe innerhalbe der Einrichtung auszurichten. Bei einer kleinen Mamma ist es hingegen möglich, mit dem Stab, dem Band und/oder der Platte das Gewebe bestmöglich zu positionieren, da die Winkelung und/oder Biegung der Untersuchungseinheiten zumindest bei sehr kleinen Mammae nicht ausreichend für die benötigte Fixierung und/oder Komprimierung ist.

Auf diese Weise wird viel Fleischgewebe in der Mitte der Untersuchungseinrichtung positioniert, wo die beste Aufnahmequalität erreichbar ist. Dadurch wird ein schönes gleichmäßiges Bild bei der Befundung bei gleichzeitig möglichst wenigen Schichten erhalten.

Eine Weiterbildung der Ausgestaltung schlägt vor, dass der Stab, das Band und/oder die Platte von der Außenseite der Patientenliege bzw. des Patienten medial nach innen zu geführt ist, dort die Mamma cranial bzw. caudal umgibt, um dann von der medialen Seite her an der Mamma anliegend zur caudalen bzw. cranialen Anlage an die Mamma zu verlaufen, wobei die Enden des Stabes, des Bandes und/oder der Platte lateral nach außen geführt und dort benachbart zueinander verlaufen.

Im Rahmen der Erfindung wurde dabei erkannt, dass bei den Untersuchungen durch den Stab, das Band und/oder die Platte die bestmögliche Fixierung und/oder Komprimierung gewährleistet wird. Gleichzeitig wird genügend Fleischgewebe in der Untersuchungseinrichtung für eine nachfolgende Befundung und/oder Gewebe- und/oder Probenentnahme mittels Biopsie eingegrenzt. Das hat zur Folge, dass mehr Fleischgewebe auch in den äußeren Schichten der Mamma eingegrenzt ist, so dass sich die Messdauer auf ein Minimum verkürzt und die Diagnose in wesentlich kürzerer Zeit als bisher gestellt werden kann.

Dazu sind in der Untersuchungseinrichtung zwei, etwa senkrecht zur Körperebene verlaufende Öffnungen und/oder Schlitze im Abstand zueinander vorgesehen, welche dem Einfädeln der beiden Enden des Stabes, des Bandes und/oder der Platte dienen. Es ist möglich, dass die Enden dieser nach außen überstehen. Auf diese Weise grenzen der Stab, das Band und/oder die Platte die Mamma im Wesentlichen konzentrisch zur Brustöffnung ein. Um die Mamma überhaupt in die Aussparung einzuführen, ist es notwendig, dass zunächst ein Abstand zwischen der Mamma und dem Stab, dem Band und/oder der Platte gegeben ist. Wird nun in axialer Richtung ein Zug und/oder Druck auf eines oder beide der Enden ausgeübt, wird die sich dazwischen befindliche Mamma in der Mitte der Untersuchungseinrichtung eingegrenzt, fixiert und/oder komprimiert. Durch weitere Einstellungen der Führung des Stabes, des Bandes und/oder der Platte ist eine noch bessere Eingrenzung der Mamma realisierbar. Die Einstellung und somit die Eingrenzung der Mamma wird solange fortgesetzt, bis ein Widerstand die flächige Anlage am Mammagewebe anzeigt und die gewünschte Kompression aufgebaut ist. Folglich ist es das Ergebnis, dass mit einer einzigen Einstellung sehr schnell eine allseitig wirksame Fixierung der Mamma in kürzester Zeit vorgenommen werden kann.

Es sind dabei verschiedene Möglichkeiten der Führung des Stabes, des Bandes und/oder der Platte denkbar, welche dem Fachmann bekannt sind.

In noch einer Ausgestaltung ist ein Andruckblock einsetzbar, der in lateraler Richtung außerhalb der Mamma angeordnet ist und welcher der endwärtigen Aufnahme des ausgehenden und auch des zurückgeführten Stabes, Bandes und/oder der Platte dient und der relativ zur Vorrichtung bewegbar ist.

Es ist weiterhin möglich, dass in axialer Richtung ein Zug und/oder Druck auf eines oder beide der Enden des Stabes, des Bandes und/oder der Platte ausgeübt wird, und zwar von lateral außerhalb des Andrucksblockes. Dabei wird die sich dazwischen befindliche Mamma in der Mitte der Untersuchungseinrichtung eingegrenzt, fixiert und/oder komprimiert. Durch weitere Einstellungen der Führung des Stabes, des Bandes und/oder der Platte ist eine noch bessere Eingrenzung der Mamma realisierbar, bis diese schließlich unter Einschluss des Andruckblockes vollständig umschlossen ist. Verständlicherweise ist es auch denkbar, dass die gewünschte Komprimierung der Mamma zusätzlich durch Bewegung des Andruckblocks in medialer Richtung erreicht wird.

Alternativ ist vorgeschlagen, dass die Stützspule von unten her senkrecht zur der durch den Stab, das Band und/oder die Platte definierten Ebene und in Richtung auf die Mamille zu bewegbar ist. Die Stützspule verhindert ein Durchhängen der Mammae durch die Untersuchungseinheit, was von den meisten Patienten als unangenehm empfunden wird. Zudem verbessert die Stützspule die Fixierung und/oder Komprimierung des Mammagewebes.

Es wird davon ausgegangen, dass die Definitionen und Ausführungen der oben genannten Begriffe für alle in dieser Beschreibung im Folgenden beschriebenen Aspekte gelten, sofern nichts anders angegeben ist.

Weitere Einzelheiten, Merkmale und Vorteile der Erfindung ergeben sich aus der nachfolgenden Beschreibung des bevorzugten Ausführungsbeispiels in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktion einander entsprechende Elemente.

Im Einzelnen zeigen:
Figur 1 eine Untersuchungseinrichtung für eine Kernresonanztomographie-Untersuchung der Mamma;
Figur 2 eine detaillierte Aufnahme der Untersuchungseinrichtung aus Figur 1; und
Figuren 3A und 3B eine isometrische Darstellung der Untersuchungseinrichtung.

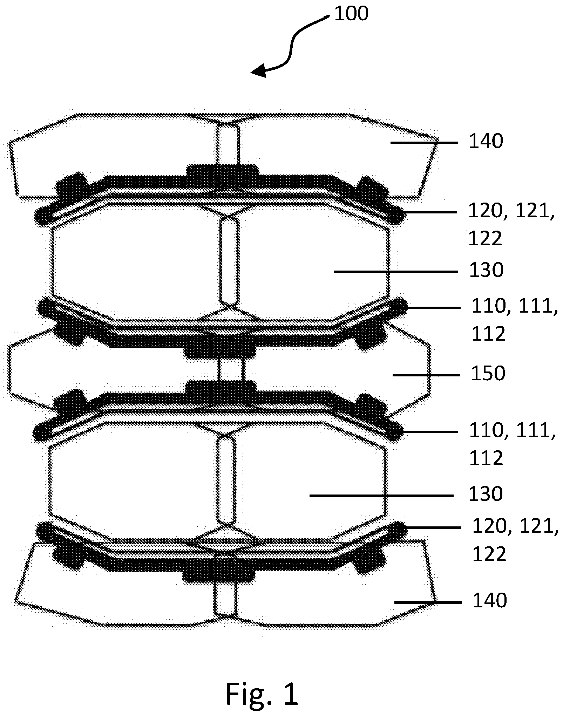

In Figur 1 ist die erfindungsgemäße Untersuchungseinrichtung (100) für eine Kernresonanztomographie-Untersuchung der Mamma eines nicht dargestellten Patienten gezeigt. Die Untersuchungseinrichtung (100) umfasst eine mediale Spule (111), eine laterale Spule (121), eine mediale Biopsieeinheit (112) und eine laterale Biopsieeinheit (122).

Dabei sind die mediale Spule (111) und die mediale Biopsieeinheit (112) miteinander verbunden und bilden eine mediale Untersuchungseinheit (110) aus, wobei diese mittig in der Untersuchungseinrichtung (100) und zwischen den Mammae angeordnet ist. Die laterale Spule (121) und die laterale Biopsieeinheit (122) sind miteinander verbunden und bilden eine laterale Untersuchungseinheit (120) aus, wobei diese seitlich in der Untersuchungseinheit (100) und der Mammae angeordnet ist.

In der Figur 1 ist es deutlich zu erkennen, dass die mediale und die laterale Untersuchungseinheit (110, 120) an die zu untersuchende Mamma angepasst sind, wobei wenigstens die sich gegenüberliegenden Enden der Spulen (111, 121) in Richtung der Mamma abgewinkelt und/oder gebogen sind. Die Winkelung und/oder Biegung der Untersuchungseinheit (110, 120) ist dabei beweglich und/oder feststellbar, sowie manuell und/oder maschinell betrieben einstellbar. Aufgrund der Winkelung und/oder Biegung passen sich die Untersuchungseinheiten (110, 120) an die natürliche Form der Mamma optimal an und umschließen sie vollständig. Dies führt zu einer ausreichenden Fixierung und/oder Komprimierung der Mamma für eine bestmögliche Bildqualität. Zudem verringert sich die Anzahl der notwendigen Aufnahmen des Mammagewebes deutlich, was nicht nur mit einer Verringerung der Strahlenbelastung des untersuchten Gewebes, sondern auch mit einer starken Verkürzung der Dauer der Mammographie einhergeht.

Die Untersuchungseinrichtung (100) kann zudem eine abnehmbare Stützspule (130) umfasst, welche von außen und unten auf die Untersuchungseinrichtung (100) aufsteckbar ist. Die aufsteckbare Stützspule (130) ist dabei Waagerecht in einem Winkel von 90° zu der Untersuchungseinheit (110, 120) angeordnet und kann in Richtung der Mamille bewegt werden. Dadurch verhindert die Stützspule zuverlässig ein Durchhängen der Mammae durch die Untersuchungseinheit (110, 120). Zudem verbessert diese die Fixierung und/oder Komprimierung des Gewebes.

Wie in Figur 1 zu erkennen ist, umfasst die Untersuchungseinrichtung (100) ein abnehmbares erstes Zusatz-Spulenelement (140), welches seitlich in Richtung der Axillae auf die Untersuchungseinrichtung (100) aufsteckbar ist. Mittels des ersten Spulenelementes (140) ist es erstmals möglich, zusammen mit der Untersuchung der Mammae auch die Axilla und die Lymphknoten der Achsel zu untersuchen. Das ist besonders wichtig, da diese meist der erste Ort sind, an dem sich bei einem positiv befundeten Mammakarzinom Metastasen bilden.

Weiterhin umfasst die Untersuchungseinrichtung (100) ein abnehmbares zweites Zusatz-Spulenelement (150), mittels welchem die medialen Untersuchungseinheiten (110) am Sternum miteinander verbindbar und/oder koppelbar sind. Dies erhöht die Stabilität der Untersuchungseinrichtung (100). Da das zweite Zusatz-Spulenelement in Richtung des Sternums des Patienten bewegbar, winkelbar, biegbar und/oder abnehmbar ausgestaltet ist, wird nicht nur eine optimale Anpassung der Untersuchungseinheit an die Form des zu untersuchenden Gewebes erreicht, sondern auch, dass der Patient auf dem zweiten Zusatz-Spulenelement abgestützt werden kann.

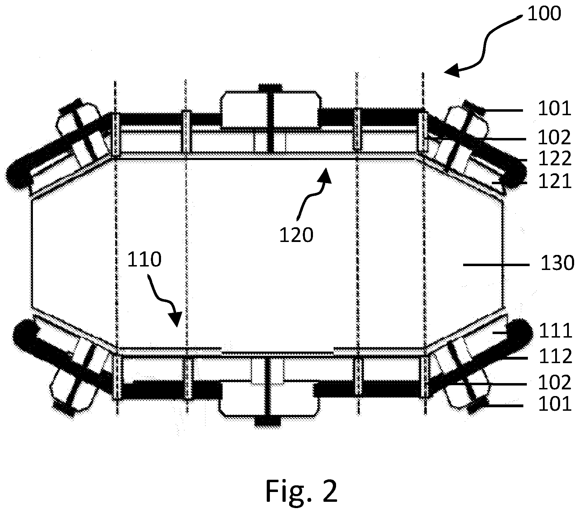

Die Figur 2 zeigt eine detaillierte Aufnahme der Untersuchungseinrichtung (100) aus Figur 1 für eine Kernresonanztomographie-Untersuchung der Mamma. Die Untersuchungseinrichtung (100) umfasst dabei eine mediale Spule (111), eine laterale Spule (121), eine mediale Biopsieeinheit (112) und eine laterale Biopsieeinheit (122). Die mediale Spule (111) und die mediale Biopsieeinheit (112) sind miteinander verbunden und bilden eine mediale Untersuchungseinheit (110) aus. Die laterale Spule (121) und die laterale Biopsieeinheit (122) sind miteinander verbunden und bilden eine laterale Untersuchungseinheit (120) aus.

In der Figur 2 ist es deutlich zu erkennen, dass die mediale und die laterale Untersuchungseinheit (110, 120) an die zu untersuchende Mamma angepasst sind, indem die sich gegenüberliegenden Enden der Spulen (111, 121) nach innen, d.h. in Richtung einer nicht dargestellten Mamma abgewinkelt und/oder gebogen sind. Die Winkelung und/oder Biegung der Untersuchungseinheit (110, 120) ist dabei beweglich und/oder feststellbar, sowie manuell und/oder maschinell betrieben einstellbar. Im Regelfall sind daher die Spulen (111, 121) und die Biopsieeinheiten (112, 122) zusammen winkelbar und/oder biegbar. Dadurch passen sich die Untersuchungseinheiten (110, 120) an die natürliche Form der Mamma optimal an, indem diese die Mamma vollständig umschließen, so dass eine ausreichende Fixierung und/oder Komprimierung der Mamma für eine bestmögliche Bildqualität erreicht wird und sich die Anzahl der notwendigen Aufnahmen des Mammagewebes deutlich reduziert.

Zudem bilden die Spulen (111, 121) und die Biopsieeinheiten (121, 122) eine bauliche Einheit aus, so dass nicht nur die Fixierung und/oder Komprimierung des Mammagewebes erreicht wird, sondern auch gleichzeitig ausreichend Gewebe für eine Befundung und/oder gegebenenfalls anschließende Biopsie vorhanden ist. Dadurch kann eine gegebenenfalls notwendige Gewebe- und/oder Probenentnahme aus der Mamma mit der gleichen hohen Qualität wie die Befundung durchgeführt werden, da die bei der Befundung mittels Kernresonanztomographie ermittelten Koordinaten einer zu entnehmenden Probe dann im nächsten Schritt direkt und unmittelbar biopsiert werden können.

Für eine anschließende Biopsie umfassen die Untersuchungseinheiten (110, 120) wenigstens einen abnehmbaren Pointer und/oder Marker (101). Der Pointer und/oder Marker (101) ist beispielsweise ein Kontrastmittel zur Markierung des untersuchten Gewebes, wobei der Pointer und/oder Marker (101) durch die Bildgebung in drei Ebenen in einem festen Winkel sichtbar ist. Auf diese Weise lässt sich optimal die Position einer Befundung für eine gegebenenfalls nachfolgende Biopsie zu markieren.

Die Untersuchungseinrichtung (100) umfasst zudem wenigstens eine Öffnung und/oder einen Schlitz (102), in welche ein steriles, medizinisches Gerät einbringbar und/oder anbringbar ist. Auf diese Weise wird erreicht, dass das zu untersuchende Gewebe in einem Winkel von ca. 90° zu den Untersuchungseinheiten (110, 120) senkrecht immer zur Öffnung und/oder dem Schlitz (102) hin frei ist. Dadurch wird das zu untersuchende Gewebe optimal in die Mitte der Vorrichtung gebracht, so die bestmögliche Aufnahme erstellt werden kann.

Die Untersuchungseinrichtung (100) weist zudem eine abnehmbare Stützspule (130) auf, welche von außen und unten auf die Untersuchungseinrichtung (100) aufsteckbar ist und Waagerecht in einem Winkel von 90° zu der Untersuchungseinheit (110, 120) angeordnet ist. Dabei kann die Stützspule (130) in Richtung der Mamille bewegt werden. Die Stützspule verhindert zuverlässig ein Durchhängen der Mammae durch die Untersuchungseinheit (110, 120) und führt somit zu einer deutlichen Verbesserung der Fixierung und/oder Komprimierung des Gewebes.

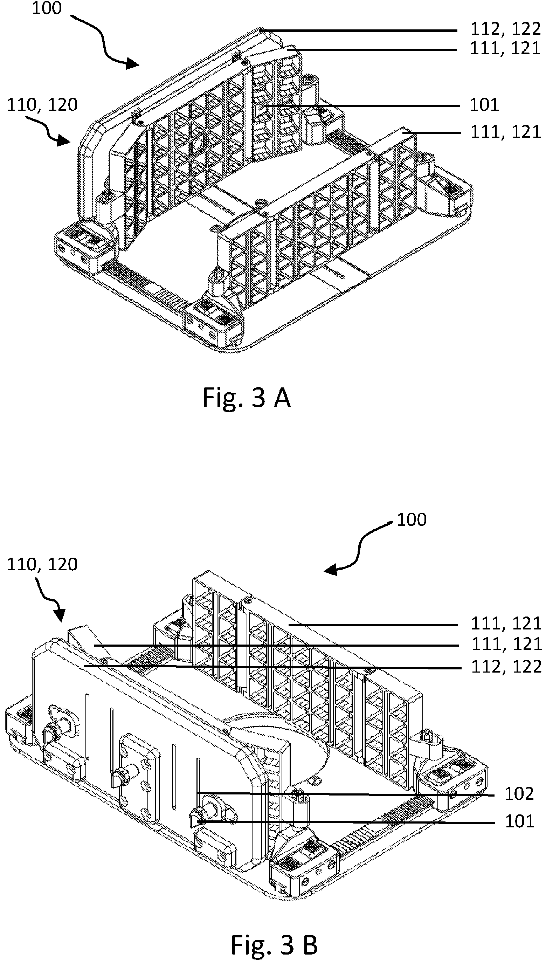

Die Figuren 3A und 3B zeigen in einer isometrischen Darstellung die erfindungsgemäße Untersuchungseinrichtung (100) für eine Kernresonanztomographie-Untersuchung der Mamma. Dabei sind die von der Untersuchungseinrichtung (100) umfasste mediale Spule (111) und die mediale Biopsieeinheit (112) miteinander verbunden und bilden eine mediale Untersuchungseinheit (110) aus. Die laterale Spule (121) und die laterale Biopsieeinheit (122) sind miteinander verbunden und bilden eine laterale Untersuchungseinheit (120) aus. Zur Vereinfachung der Darstellung ist lediglich eine Untersuchungseinheit (110, 120) vollständig dargestellt.

Die mediale und die laterale Untersuchungseinheit (110, 120) sind aufgrund einer Winkelung und/oder Biegung der sich gegenüberliegenden Enden der Spulen (111, 121) nach innen an die zu untersuchende und hier nicht dargestellte Mamma angepasst. Die Winkelung und/oder Biegung der Untersuchungseinheit (110, 120) ist dabei beweglich und/oder feststellbar, sowie manuell und/oder maschinell betrieben einstellbar.

Wie in den Figuren 3A und 3B deutlich zu erkennen ist, sind die Spule (111, 121) und die Biopsieeinheit (112, 122) zwar miteinander verbunden und bilden eine Einheit aus, allerdings ist nur die Spule (111, 121) gewinkelt und/oder gebogen. Die Biopsieeinheit (112, 122) ist dabei nicht durch Winkelung und/oder Biegung an die natürliche Form der Mamma angepasst. Dennoch wird eine optimale Fixierung und/oder Komprimierung der Mamma für eine bestmögliche Bildqualität erreicht, so dass sich die Anzahl der notwendigen Aufnahmen des Mammagewebes und die Dauer der Untersuchung, also der Mammographie, deutlich reduzieren.

Um gleichzeitig mit der Befundung auch ausreichend Gewebe für eine gegebenenfalls anschließende Biopsie zu fixieren und/oder zu komprimieren, weist die Untersuchungseinheit (110, 120) wenigstens einen abnehmbaren Pointer und/oder Marker (101) auf. Der Pointer und/oder Marker (101) ist beispielsweise ein Kontrastmittel zur Markierung des untersuchten Gewebes, wobei der Pointer und/oder Marker (101) durch die Bildgebung in drei Ebenen in einem festen Winkel sichtbar ist.

Des Weiteren umfasst die Untersuchungseinrichtung (100) wenigstens eine Öffnung und/oder einen Schlitz (102), in welche ein steriles, medizinisches Gerät ein- und/oder angebracht werden kann. Dadurch wird das zu untersuchende Gewebe noch besser fixiert und/oder komprimiert, sowie in die Mitte der Vorrichtung angeordnet. An dieser Stelle ist eine bestmögliche Bildqualität erreichbar.

Zudem ist den Figuren 3A und 3B deutlich zu entnehmen, dass die mediale und/oder laterale Untersuchungseinheit (110, 120), insbesondere deren Aufnahme und/oder Führung, in der Position drehbar und/oder einstellbar ist. Das bedeutet, dass jede Untersuchungseinheit (110, 120) unabhängig von der anderen um bis zu 360° drehbar und/oder in alle Richtungen stufenlos einstellbar ist. Auf diese Weise wird eine optimale Anpassung an die Form der entsprechenden Mamma erreicht und somit eine bestmögliche Fixierung und/oder Komprimierung der Mamma sichergestellt.

### Bezugszeichenliste

- 100: Untersuchungseinrichtung
- 101: Pointer und/oder Marker
- 102: Öffnung und/oder einen Schlitz

- 110: mediale Untersuchungseinheit
- 111: mediale Spule
- 112: mediale Biopsieeinheit

- 120: laterale Untersuchungseinheit
- 121: laterale Spule
- 122: laterale Biopsieeinheit

- 130: Stützspule

- 140: erstes Zusatz-Spulenelement

- 150: zweites Zusatz-Spulenelement

## Patentansprüche

1. Patientenliege für eine Kernresonanztomographie-Untersuchung eines Körperteils eines Patienten, insbesondere der Mamma, umfassend zwei Aussparungen, welche jeweils für die Aufnahme einer Mamma bestimmt sind, und eine in deren unmittelbarer Nähe angeordnete Untersuchungseinrichtung (100), wobei
- die Untersuchungseinrichtung (100) unterhalb der Patientenliege angeordnet ist und eine mediale Spule (111), eine laterale Spule (121), eine mediale Biopsieeinheit (112) und eine laterale Biopsieeinheit (122) umfasst, wobei die Spulen (111, 121) und die Biopsieeinheiten (112, 122) senkrecht zur Körperoberfläche des Patienten ausgerichtet sind;
- die mediale Spule (111) und die mediale Biopsieeinheit (112) verbunden sind, miteinander in Wirkverbindung stehen und eine mediale Untersuchungseinheit (110) ausbilden, wobei die mediale Spule (111) Kontakt mit der Mamma hat;
- die laterale Spule (121) und die laterale Biopsieeinheit (122) verbunden sind, miteinander in Wirkverbindung stehen und eine laterale Untersuchungseinheit (120) ausbilden, wobei die laterale Spule (121) Kontakt mit der Mamma hat; und
- die sich gegenüberliegenden Enden der medialen Untersuchungseinheit (110) und die sich gegenüberliegenden Enden der lateralen Untersuchungseinheit (120) jeweils in Richtung der zu untersuchenden Mamma abgewinkelt und/oder gebogen sind, **dadurch gekennzeichnet, dass**
die Winkelung und/oder Biegung der medialen und/oder lateralen Untersuchungseinheit (110, 120) beweglich und optional feststellbar ist, wobei die Winkelung und/oder Biegung der Untersuchungseinheiten (110, 120) 1° bis 50° beträgt.

2. Patientenliege nach dem vorhergehenden Anspruch, **dadurch gekennzeichnet, dass** die Winkelung und/oder Biegung der medialen und/oder lateralen Untersuchungseinheit (110, 120) manuell und/oder maschinell betrieben, insbesondere mechanisch, pneumatisch und/oder elektrisch, einstellbar ist.

3. Patientenliege nach einem der vorhergehenden Ansprüche, **dadurch gekennzeichnet, dass** die mediale und/oder laterale Untersuchungseinheit (110, 120) wenigstens einen Pointer und/oder Marker (101) umfasst.

4. Patientenliege nach einem der vorhergehenden Ansprüche, **dadurch gekennzeichnet, dass** die mediale und/oder laterale Untersuchungseinheit (110, 120), insbesondere deren Aufnahme und/oder Führung, in der Position drehbar und/oder einstellbar ist.

5. Patientenliege nach einem der vorhergehenden Ansprüche, **dadurch gekennzeichnet, dass** die Untersuchungseinrichtung (100) eine Stützspule (130) umfasst, welche von außen und unten auf die Untersuchungseinrichtung (100) aufsteckbar ist.

6. Patientenliege nach dem vorhergehenden Anspruch, **dadurch gekennzeichnet, dass** die Stützspule (130) senkrecht in Richtung der Patientenliege und in Richtung auf die Mamille zu bewegbar und/oder abnehmbar ist.

7. Patientenliege nach einem der vorhergehenden Ansprüche,
**dadurch gekennzeichnet, dass** die Untersuchungseinrichtung (100) eine Ringspule umfasst, welche von außen auf die Untersuchungseinrichtung (100) aufsteckbar ist, horizontal am Körper des Patienten anliegt und sich in Richtung dessen Kopfes erstreckt.

8. Patientenliege nach einem der vorhergehenden Ansprüche,
**dadurch gekennzeichnet, dass** die Untersuchungseinrichtung (100) ein erstes Zusatz-Spulenelement (140) umfasst, welches seitlich in Richtung der Axillae auf die Untersuchungseinrichtung (100) aufsteckbar ist.

9. Patientenliege nach dem vorhergehenden Anspruch, **dadurch gekennzeichnet, dass** die Ringspule und/oder das erstes Zusatz-Spulenelement (140) abnehmbar ist.

10. Patientenliege nach einem der vorhergehenden Ansprüche,
**dadurch gekennzeichnet, dass** die Untersuchungseinrichtung (100) ein zweites Zusatz-Spulenelement (150) umfasst, mittels welchem die medialen Untersuchungseinheiten (110) am Sternum miteinander verbindbar und/oder koppelbar sind.

11. Patientenliege nach dem vorhergehenden Anspruch, **dadurch gekennzeichnet, dass** das zweite Zusatz-Spulenelement (150) in Richtung des Sternums des Patienten bewegbar, winkelbar, biegbar und/oder abnehmbar ausgestaltet ist.

12. Patientenliege nach einem der vorhergehenden Ansprüche,
**dadurch gekennzeichnet, dass** die Untersuchungseinrichtung (100) wenigstens eine Öffnung und/oder einen Schlitz (102) umfasst, in welche ein Stab, ein Band und/oder eine Platte einbringbar und/oder anbringbar ist.

13. Patientenliege nach dem vorhergehenden Anspruch, **dadurch gekennzeichnet, dass** der Stab, das Band und/oder die Platte von der Außenseite der Patientenliege bzw. des Patienten medial nach innen zu geführt ist, dort die Mamma cranial bzw. caudal umgibt, um dann von der medialen Seite her an der Mamma anliegend zur caudalen bzw. cranialen Anlage an die Mamma zu verlaufen, wobei die Enden des Stabes, des Bandes und/oder der Platte lateral nach außen geführt und dort benachbart zueinander verlaufen.

14. Patientenliege nach einem der vorhergehenden Ansprüche,
**dadurch gekennzeichnet, dass** ein Andruckblock einsetzbar ist, der in lateraler Richtung außerhalb der Mamma angeordnet ist und welcher der endwärtigen Aufnahme des ausgehenden und auch des zurückgeführten Stabes, Bandes und/oder der Platte dient und der relativ zur Untersuchungseinrichtung (100) bewegbar ist.

15. Patientenliege nach einem der vorhergehenden Ansprüche,
**dadurch gekennzeichnet, dass** die Stützspule (130) von unten her senkrecht zur der durch den Stab, das Band und/oder die Platte definierten Ebene und in Richtung auf die Mamille zu bewegbar ist.

## Claims

1. Patient table for a nuclear magnetic resonance tomography examination of a body part of a patient, in particular of the mammary, comprising two recesses, which are in each case intended to receive a mammary and an examination device (100), which is disposed in the direct vicinity thereof, wherein
- The examination device (100) is disposed below the patient table and comprises a medial coil (111), a lateral coil (121), a medial biopsy unit (112) and a lateral biopsy unit (122), wherein the coils (111; 121) and the biopsy units (112, 122) are oriented perpendicular to the body surface of the patient;
- the medial coil (111) and the medial biopsy unit (112) are connected, are in operative connection with one another and form a medial examination unit (110), the medial coil (111) being in contact with the mammary;
- the medial coil (121) and the medial biopsy unit (122) are connected, are in operative connection with one another and form a medial examination unit (120), the medial coil (121) being in contact with the mammary, and
- the mutually opposite ends of the medial examination unit (110) and the mutually opposite ends of the lateral examination unit (120) are in each case angled and/or curved in the direction of the mammary to be examined,
**characterized in that**
the angling or curvature of the medial and/or lateral examination unit (110, 120) is movable and optionally fixable, the angling and/or curvature of the examination units (110, 120) being 1° to 50°.

2. Patient table according to the previous claim,
**characterised in that** the angling and/or curvature of the medial and/or lateral examination unit (110, 120) is manually or machine operated, in particularly mechanically, pneumatically and/or electrically adjustable.

3. Patient table according to one of the previous claims,
**characterised in that** the medial and/or lateral examination unit (110, 120) comprises at least one pointer and/or marker (101).

4. Patient table according to one of the previous claims,
**characterised in that** the medial and/or lateral examination unit (110, 120), in particular the receptacle and/or guide thereof, is rotatable and/or adjustable in position.

5. Patient table according to one of the previous claims,
**characterised in that** the examination device (100) comprises a supporting coil (130), which can be plugged from the outside and from below onto the examination device (100).

6. Patient table according to the previous claim,
**characterised in that** the supporting coil (130) can be moved and/or removed perpendicularly in the direction of the patient table and in the direction towards the mammary.

7. Patient table according to one of the previous claims,
**characterised in that** the examination device (100) comprises a ring coil, which can be plugged from the outside onto the examination device (100), bears horizontally against the body of the patient and extends in the direction of the patient's head.

8. Patient table according to one of the previous claims,
**characterised in that** the examination device (100) comprises a first additional coil element (140), which can be plugged laterally in the direction of the axillae onto the examination device (100).

9. Patient table according to the previous claims,
**characterised in that** the ring col and/or the first additional coil element (140) is removable.

10. Patient table according to one of the previous claims,
**characterised in that** the examination device (100) comprises a second additional coil element (150), by means of which the medial examination units (110) on the sternum can be connected and or coupled to one another.

11. Patient table according to the previous claim,
**characterised in that** the second additional coil element (150) is designed so as to be movable, angleable, bendable and/or removable in the direction of the patient's sternum.

12. Patient table according to one of the previous claims,
**characterised in that** the examination device (100) comprises at least one opening and/or a slit (102) into which a rod, a tape and/or a plate can be introduced and/or attached.

13. Patient table according to the previous claim,
**characterised in that** the road, the band and/or the plate is guided medially inwardly from the outside of the patient table or of the patient, here surrounds the breast cranially or caudally, and then runs from the medial side, bearing against the mammary for caudal or cranial contact with the breast, the ends of the rod, the band and/or the plate being guided laterally outwardly, where they run adjacent to one another.

14. Patient table according to one of the preceding claims,
**characterised in that** a pressure block can be used which is disposed in a lateral direction outside the mammary and which serves for the reception of the outgoing and also of the led-back rod, band and/or plate, at its ends and which can be moved relative to the examination equipment (100).

15. Patient table according to one of the preceding claims,
**characterised in that** the supporting table (130) is mounted from below perpendicular to the plane defined by the rod, band and/or plate and towards the breast.

## Revendications

1. Table d'examen médical destinée à l'examen de tomographie de résonance magnétique nucléaire du membre d'un patient, notamment du sein, comprenant deux réservations dont chacune est destinée à réceptionner un sein, et un dispositif d'examen (100) placé à proximité immédiate, sachant que
- le dispositif d'examen (100) est disposé en dessous de la table d'examen médical et comprend une bobine médiane (111), une bobine latérale (121), une unité de biopsie médiane (112) et une unité de biopsie latérale (122), sachant que les bobines (111, 121) et les unités de biopsie (112, 122) sont disposées verticalement par rapport à la surface du corps du patient ;
- la bobine médiane (111) et l'unité de biopsie médiane (112) sont reliées, sont connectées activement et forment une unité d'examen médiane (110), sachant que la bobine médiane (111) est en contact avec le sein ;
- la bobine latérale (121) et l'unité de biopsie latérale (122) sont reliées, sont connectées activement et forment une unité d'examen latérale (120), sachant que la bobine latérale (121) est en contact avec le sein ; et
- que les extrémités se faisant face de l'unité d'examen médiane (110) et les extrémités se faisant face de l'unité d'examen latérale (120) sont chacune courbées et/ou coudées dans le sens du sein à examiner,
**caractérisée par le fait que**
l'angle et/ou la courbe de l'unité d'examen médiane et/ou latérale (110, 120) est mobile et peut être en option arrêté, sachant que l'angle et/ou la courbe des unités d'examen (110, 120) est de 1° à 50°.

2. Table d'examen médical selon la revendication précédente,
**caractérisée par le fait que** l'angle et/ou la courbe de l'unité d'examen médiane et/ou latérale (110, 120) peut être réglé manuellement et/ou par une machine, notamment mécaniquement, pneumatiquement et/ou électriquement.

3. Table d'examen médical selon une des revendications précédentes,
**caractérisée par le fait que** l'unité d'examen médiane et/ou latérale (110, 120) comprend au moins un pointeur et/ou un marqueur (101).

4. Table d'examen médical selon une des revendications précédentes,
**caractérisée par le fait que** l'unité d'examen médiane et/ou latérale (110, 120), notamment sa réception et/ou guidage peut être tournée et/ou réglée dans sa position.

5. Table d'examen médical selon une des revendications précédentes,
**caractérisée par le fait que** le dispositif d'examen (100) comprend une bobine de support (130), qui peut être enfichée de l'extérieur et en bas sur le dispositif d'examen (100).

6. Table d'examen médical selon la revendication précédente,
**caractérisée par le fait que** la bobine de support (130) peut être déplacée et/ou retirée verticalement en direction de la table d'examen médical et en direction du sein.

7. Table d'examen médical selon une des revendications précédentes,
**caractérisée par le fait que** l'unité d'examen (100) comprend un tore qui peut être enfiché de l'extérieur sur le dispositif d'examen (100), repose horizontalement sur le corps du patient et s'étend en direction de la tête de ce dernier.

8. Table d'examen médical selon une des revendications précédentes,
**caractérisée par le fait que** le dispositif d'examen (100) comprend un premier élément de bobine supplémentaire (140), qui peut être enfiché latéralement en direction de l'aisselle sur le dispositif d'examen (100).

9. Table d'examen médical selon la revendication précédente,
**caractérisée par le fait que** le tore et/ou le premier élément de bobine supplémentaire (140) est amovible.

10. Table d'examen médical selon une des revendications précédentes,
**caractérisée par le fait que** le dispositif d'examen (100) comprend un deuxième élément de bobine supplémentaire (150), qui permet de relier et/ou de coupler entre elles les unités d'examen médianes (110) sur le sternum.

11. Table d'examen médical selon la revendication précédente,
**caractérisée par le fait que** le deuxième élément de bobine supplémentaire (150) est conçu de manière à pouvoir être déplacé, former un angle, fléchi et/ou retiré en direction du sternum du patient.

12. Table d'examen médical selon une des revendications précédentes,
**caractérisée par le fait que** le dispositif d'examen (100) comprend au moins une ouverture et/ou une fente (102) dans laquelle on peut enficher ou mettre une tige, une bande et/ou une plaque.

13. Table d'examen médical selon la revendication précédente,
**caractérisée par le fait que** la tige, la bande et/ou la plaque est insérée depuis l'extérieur de la table d'examen médical et du patient, de façon médiane vers l'intérieur, entoure là le sein de façon crâniale ou caudale, pour suivre ensuite un parcours partant du côté médian en reposant sur le sein vers la position caudale ou crâniale, sachant que les extrémités de la tige, la bande et/ou la plaque sont guidées latéralement vers l'extérieur et suivent là un parcours voisin les unes des autres.

14. Table d'examen médical selon une des revendications précédentes,
**caractérisée par le fait qu'**il est possible d'employer un bloc de pression, qui est disposé dans le sens latéral en dehors du sein et qui sert à la réception finale de la tige, de la bande et/ou de la plaque sortante et également amenée, et qui peut être déplacé par rapport au dispositif d'examen (100).

15. Table d'examen médical selon une des revendications précédentes,
**caractérisée par le fait que** la bobine de support (130) peut être déplacée verticalement depuis le bas vers le niveau défini par la tige, la bande et/ou la plaque et en direction du sein.
